# EUROPÄISCHE PATENTSCHRIFT

(11) **EP 1 152 685 B1**
(45) Veröffentlichungstag und Bekanntmachung des Hinweises auf die Patenterteilung: **29.10.2003**
(21) Anmeldenummer: 00907567.2
(22) Anmeldetag: 14.02.2000
(51) Int. Cl.: A61B 1/267, A61B 1/00

(54) **VERFORMBARES FIBROSKOP MIT VERSCHIEBBAREM ZUSATZGERÄT**
DEFORMABLE FIBERSCOPE WITH A DISPLACEABLE SUPPLEMENTARY DEVICE
FIBROSCOPE DEFORMABLE AVEC APPAREIL AUXILIAIRE AJUSTABLE

(30) Priorität: 15.02.1999 DE 19906191
(43) Veröffentlichungstag der Anmeldung: 14.11.2001
(73) Patentinhaber: Herrmann, Ingo F., 81479 München (DE)
(72) Erfinder: Herrmann, Ingo F., 81479 München (DE)
(74) Vertreter: Manitz, Finsterwald & Partner GbR
(86) Internationale Anmeldenummer: EP0001200
(87) Internationale Veröffentlichungsnummer: WO00048506

(56) Entgegenhaltungen:
- WO-A-97/41767
- WO-A-99/27840
- US-A- 1 891 054
- US-A- 5 154 164
- US-A- 5 643 175
- US-A- 5 904 648

## Beschreibung

Die Erfindung betrifft ein verformbares Endoskop, das einen oder mehrere Licht-/Bildübertragungskanäle und wenigstens ein Zusatzgerät aufweist.

Beispielsweise sind Gastroskope bekannt, die dem Patienten für eine Magenspiegelung über den Mund in Speiseröhre und Magen eingeführt werden. Derartige Gastroskope besitzen einen zentralen Arbeitskanal, in den als Zusatzgerät beispielsweise eine kleine Biopsie-Zange eingeführt wird, um unter Beobachtung über den Licht-/Bildübertragungskanal eine Gewebeprobe aus dem Magen oder aus der Speiseröhre zu entnehmen.

Nachteilig an diesen bekannten Endoskopen ist, daß ihre Anwendungsmöglichkeiten in unerwünschtem Maße begrenzt sind und ihre Anwendung für den Patienten eine Belastung bedeutet. Insbesondere ist für das Einführen bekannter Endoskope die vorherige medikamentöse Dämpfung erforderlich. Eine unbeabsichtigte Verletzung des Patienten, beispielsweise der Rachen-Schleimhaut durch ein oral eingeführtes Gastroskop, kann nicht immer vermieden werden.

Die US-A-5643175 beschreibt ein Endoskop gemäß dem Oberbegriff des Anspruchs 1.

Die WO 97/41767 beschreibt einen Befestigungsmechanismus zur Befestigung eines Videoendoskops und eines chirurgischen Instruments aneinander.

Aus der US-A-1891054 ist ein Endoskop mit einer Biopsie-Schlinge bekannt, die innerhalb eines seitlich befestigten Rohres geführt ist.

Es ist eine Aufgabe der Erfindung, ein Endoskop zu schaffen, das bei einer geringeren Verletzungsgefahr und einer angenehmeren subjektiven Wahrnehmung durch den Patienten ein breiteres Anwendungsspektrum bietet als bekannte Endoskope.

Diese Aufgabe wird durch die Merkmale des Anspruchs 1 gelöst.

Die Erfindung löst sich somit von der seit langem eingefahrenen Ansicht, daß Endoskope mit einem im wesentlichen kreisrunden Querschnitt zu versehen seien. Der Querschnitt des Einführabschnitts des erfindungsgemäßen Endoskops ist nicht rotationssymmetrisch, so daß das Endoskop den vorgesehenen Anwendungen besser angepaßt werden kann.

Vorzugsweise ist der Querschnitt des Einführabschnitts an den Querschnitt der Körperöffnung angepaßt, in die das Endoskop eingeführt werden soll und die im allgemeinen einen nicht runden Querschnitt besitzt. Dadurch kann die Raumausnutzung, insbesondere an der für das Einführen des Endoskops engsten Körperstelle, optimiert werden. Dies führt zu einer angenehmeren subjektiven Wahrnehmung seitens des Patienten, und im Körper des Patienten kann mehr Raum für die gewünschte Diagnose- oder Therapiemaßnahme bereitstehen. Ein besonderer Vorteil einer derartigen Querschnittsanpassung besteht darin, daß in vielen Anwendungsfällen auf medikamentöse Dämpfung, Relaxation oder Betäubung verzichtet werden kann, da aufgrund der optimierten Querschnitts- bzw.

Raumausnutzung Abwehrreflexe des Patienten leichter vermieden werden können.

Indem der Querschnitt des Endoskops eine nicht runde Form besitzt, kann außerdem eine vorteilhafte Stabilität des eingeführten Endoskops gegenüber unerwünschten Verdrehungen bezüglich seiner Längsrichtung erzielt werden. Bei dem runden Querschnitt bekannter Endoskope kann es im Laufe des Einführvorganges zu einer unerwünschten Verdrehung des Endoskops kommen. Falls dagegen das Endoskop - wie es die Erfindung vorsieht - im Querschnitt asymmetrisch ausgebildet ist, kann die Form der Körperöffnung, in welche das Endoskop eingeführt ist, ausgenutzt werden, um eine Verdrehung des eingeführten Endoskops zu blokkieren. Dies erleichtert das weitere Einführen des Endoskops und verringert die Verletzungsgefahr.

Der nicht runde Querschnitt des Endoskops kann dadurch verwirklicht werden, daß das Zusatzgerät - entgegen dem Aufbau bekannter Endoskope - an nicht zentraler Lage innerhalb des Querschnitts des Einführabschnitts angeordnet ist. Insbesondere kann ein für die Aufnahme des Zusatzgeräts vorgesehener Arbeitskanal an nicht zentraler Lage, sondern bezüglich der Längsachse des Endoskops in seitlicher, exzentrischer Anordnung vorgesehen sein.

Der nicht runde Querschnitt des Endoskops kann auch dadurch erzielt werden, daß die Abmessung des Querschnitts seines Einführabschnitts in einer Richtung deutlich größer ist als die Abmessung in einer hierzu orthogonalen Richtung, zum Beispiel um einen Faktor von mindestens 1,5. Der Querschnitt kann beispielsweise näherungsweise einem gleichschenkligen Dreieck oder einem spiegelsymmetrischen Trapez entsprechen, dessen Ecken jeweils abgerundet sind.

In einer bevorzugten Ausführungsform ist das Endoskop als Pharingoösophagogastroskop ausgebildet zur Untersuchung von Rachen (Pharynx), Speiseröhre (Ösophagus) oder Magen des Patienten, indem der Querschnitt des Einführabschnitts des gesamten Endoskops an den Querschnitt eines Nasengangs angepaßt ist. Ein derartiges Endoskop kann - im Gegensatz zur herkömmlichen Gastroskopie - dem Patienten über einen Nasengang in den Rachen und die Speiseröhre eingeführt werden.

Das erfindungsgemäße Pharingoösophagogastroskop bietet u.a. folgende Vorteile:
a) Bei dem nasalen Einführen in den Rachen und in die Speiseröhre kann aufgrund der gleichzeitigen optischen Überwachung über den Licht-/Bildübertragungskanal und aufgrund der Verformbarkeit des Pharingoösophagogastroskops eine Verletzung des Patienten, insbesondere der Rachenschleimhäute, besonders leicht vermieden werden.
b) Im Gegensatz zu dem oralen Einführen eines bekannten Gastroskops erzeugt das nasale Einführen des erfindungsgemäßen Endoskops beim Patienten keinen Abwehrreflexe, insbesondere keine Beißreflexe.
c) Im Unterschied zur herkömmlichen Gastroskopie ist keine lokale medikamentöse Dämpfung des Kehlkopfbereiches erforderlich, so daß der Patient durch die Anwendung des erfindungsgemäßen Endoskops insofern nicht beeinträchtigt wird und nach der Anwendung die ärztliche Praxis oder die Klinik problemlos verlassen kann, ohne auf das Abklingen einer Dämpfungsmaßnahme warten zu müssen.
d) Bei der Anwendung des erfindungsgemäßen des Pharingoösophagogastroskops ist auch ein geringerer Personalaufwand erforderlich, da auf die Hilfestellung durch eine Schwester und einen Springer (zweite Schwester oder Pfleger) verzichtet werden kann und auch kein Anästhesist erforderlich ist. Die Anwendung des Endoskops kann durch einen einzigen Arzt mit seiner Sprechstundenhilfe erfolgen. Ein eigens eingerichteter Endoskopieraum (Operationssaal) ist nicht erforderlich. Die Anwendung des Pharingoösophagogastroskops erfolgt vielmehr auf einem normalen Untersuchungsstuhl in der ärztlichen Praxis. Dies reduziert die Anwendungskosten natürlich in erheblichem Maße.
e) Das erfindungsgemäße Pharingoösophagogastroskop wird aufgrund seines optimierten Querschnitts und seiner somit vergleichsweise geringen Querschnittsfläche vom Patienten als angenehmer empfunden als ein herkömmliches Gastroskop, das üblicherweise einen kreisrunden Querschnitt von ca. 11 mm besitzt und dabei unnötigen Totraum belegt. Zu dieser angenehmeren Wahrnehmung trägt auch bei, daß das Pharingoösophagogastroskop nasal eingeführt wird, so daß der vergleichsweise sensible Mundraum frei von Fremdkörpern bleibt.
f) Das durch einen einzigen Nasengang eingeführte Pharingoösophagogastroskop ermöglicht es dem Patienten, weiterhin sowohl durch den Mund als auch durch die Nase, nämlich durch den nicht belegten Nasengang zu atmen.
g) Das nasal in den Rachenraum eingeführte Pharingoösophagogastroskop kann auf besonders einfache, angenehme und ungefährliche Weise in die Speiseröhre weitergeführt werden, wenn der Patient gleichzeitig eine Flüssigkeit, beispielsweise Wasser, trinkt. Dies ist aufgrund des freien Mundraums problemlos möglich. Der Trinkvorgang kann über den Licht-/Bildübertragungskanal vom Arzt verfolgt werden. Bei einem Schluckvorgang wird das Pharingoösophagogastroskop am Kehlkopf und an der Speiseröhren-Schließmuskulatur vorbei in die Speiseröhre geführt.
h) Das nasal eingeführte Endoskop ermöglicht ein realitätsnahes Studium der Schluckmotorik, der Kontraktionsbewegungen der Speiseröhrenmuskulatur (Peristaltik) und der zugeordneten Schließmuskel (Sphinkter). Diese Untersuchungen können unter Zuhilfenahme des Zusatzgerätes und unter gleichzeitiger Beobachtung über den Licht-/Bildübertragungskanal erfolgen. Solche Funktionsanalysen können sowohl beim Trinken von Flüssigkeit, als auch bei Einnahme von fester Nahrung erfolgen. Es zeigt sich eine geringere Verfälschung des Untersuchungsergebnisses als bei bekannten Gastroskopen, da der Mundbereich von dem nasal eingeführten Pharingoösophagogastroskop nicht blockiert ist und da das Pharingoösophagogastroskop aufgrund seines optimierten Querschnitts eine geringere und somit weniger störende Querschnittsfläche aufweist.
i) Die Anwendung des erfindunsgemäßen Endoskops ist nicht auf Diagnostizierverfahren beschränkt, sondern es eröffnen sich neue Möglichkeiten des Studiums des menschlichen oder tierischen Körpers. Beispielsweise ermöglicht das dem Nasengang angepaßte Pharingoösophagogastroskop die Erforschung des neuen Studiengebietes der sogenannten Somnoskopie:
   Da das nasal eingeführte Endoskop dem Patienten weiterhin die Möglichkeit bietet, durch Mund oder Nase zu atmen, und da es vom Patienten auch nicht als besonders unangenehm empfunden wird, kann das Endoskop ohne weiteres über Nacht im Körper des Patienten belassen werden, um über mehrere Stunden andauernde Studien durchzuführen. Beispielsweise kann als Zusatzgerät des erfindungsgemäßen Endoskops eine pH-Meßsonde verwendet werden, die über Nacht an mehreren Stellen entlang der Speiseröhre sowie im Magen den pH-Wert bestimmt, um zu überprüfen, ob bei vergleichsweise ungestörten und stationären Bedingungen sich unerwartete lokale oder zeitliche Anomalien ergeben. Selbstverständlich bleibt bei einer derartigen Anwendung die Möglichkeit einer optischen Kontrolle über den Licht-/Bildübertragungskanal erhalten.

Bei der Ausbildung des Endoskops als Pharingoösophagogastroskop ist es bevorzugt, wenn die Basislänge der Querschnitts-Grundform, also beispielsweise der erläuterten Dreiecks- oder Trapezform, höchstens 3,5 mm beträgt. Ausführliche Studien haben nämlich ergeben, daß bei einer derartigen Abmessung das Einführen des Endoskops in einen Nasengang prinzipiell bei allen Patienten noch möglich ist. Außerdem ist bevorzugt, wenn der maximale Querschnitt des jeweiligen Zusatzgeräts höchstens ca. 3 mm, insbesondere maximal ca. 2 mm beträgt, damit das Zusatzgerät zusammen mit dem restlichen Pharingoösophagogastroskop bei jedem Patienten problemlos durch einen Nasengang geführt werden kann. Der Querschnitt des Zusatzgeräts kann jedoch auch dem individuellen maximalen Querschnitt des Nasengangs des Patienten angepaßt werden. Die typische Gesamtlänge des Pharingoösophagogastroskops beträgt beispielsweise 76 cm.

Die Erfindung kann auch in einem Tracheobronchoskop zur Untersuchung der Luftröhre und der Luftröhrenäste verwirklicht sein. Die vorstehend erläuterten Vorteile des Pharingoösophagogastroskops kommen auch bei einem solchen Tracheobronchoskop zur Geltung.

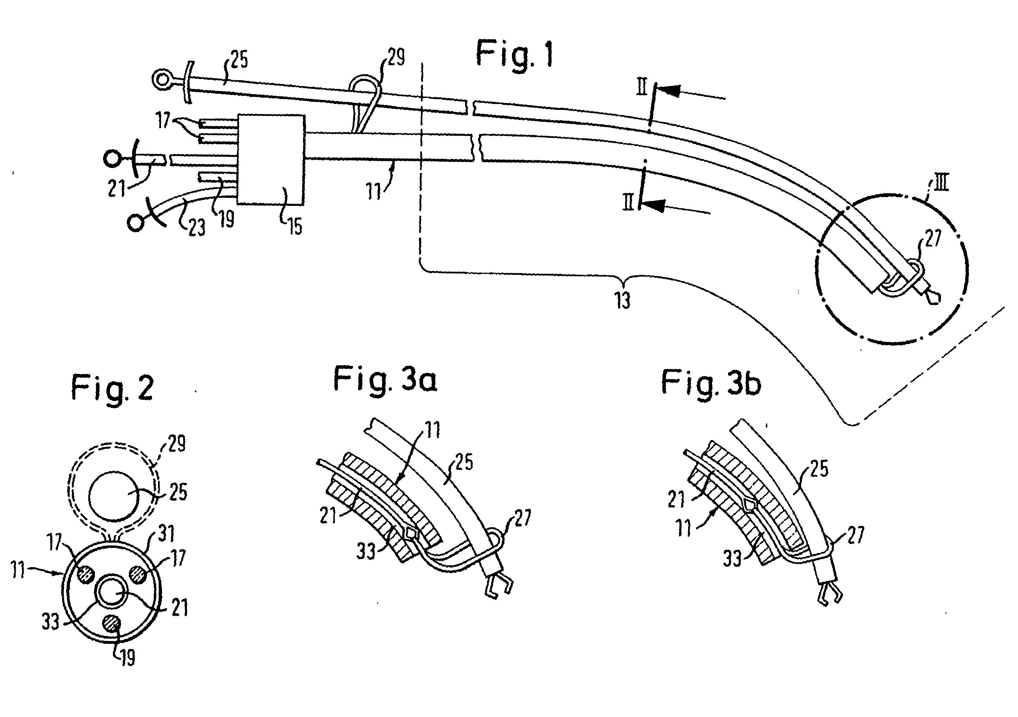

Das erfindungsgemäße Endoskop ist aus mehreren Teilen aufgebaut, nämlich aus einem Fibroskopteil, der als eine geschlossene Einheit den bzw. die Licht-/Bildübertragungskanäle sowie vorzugsweise einen oder mehrere Arbeitskanäle enthält, sowie aus dem einen oder den mehreren Zusatzgeräten. Da das Zusatzgerät dem Fibroskopteil gleichsam aufgesetzt werden kann, wird diese Ausführungsform auch als "Huckepack-System" bezeichnet (vgl. Fig. 1). Das Zusatzgerät befindet sich hierbei vorzugsweise außerhalb des eigentlichen Fibroskopteils.

In Abhängigkeit von der Querschnittsfläche und -form der Körperöffnung, beispielsweise des Nasengangs, kann das Zusatzgerät dem Fibroskopteil oder umgekehrt der Fibroskopteil dem Zusatzgerät aufgesetzt werden, und das Zusatzgerät kann einen kleineren oder einen größeren Querschnitt aufweisen als der Fibroskopteil.

Der Fibroskopteil und das bzw. die Zusatzgeräte können entlang ihrer jeweiligen Längsrichtung relativ zueinander verschoben werden (sogenanntes "Shuttle-System"). Dadurch ergibt sich eine flexible Handhabung und ein variabler Aktionsradius des betreffenden Zusatzgerätes.

Beispielsweise kann bei der Verwendung einer Biopsie-Zange als Zusatzgerät eine Verschiebbarkeit von ca. 5 cm ausreichen, um einerseits zur Vermeidung einer Behinderung der Beobachtung des Einführvorganges über den Licht-/Bildübertragungskanal die Biopsie-Zange bezüglich des Fibroskopteils zurückzuziehen, und um andererseits die Biopsie-Zange relativ zum stationären Fibroskopteil in distaler Richtung auszufahren zum Zwecke einer Probenentnahme.

Eine relative Verschiebbarkeit von ca. 35 cm kann sich insbesondere bei Langzeit-Untersuchungen als vorteilhaft erweisen, bei denen bei stationärem Zusatzgerät, beispielsweise pH-Meßsonde, lediglich der Fibroskopteil verschoben werden soll, um an einer bestimmten pH-Meßstelle eine optische Beobachtung durchzuführen, ohne hierbei die andauernde Messung mit der pH-Meßsonde zu stören oder zu verfälschen.

Um eine Verbindung des Fibroskopteils und des bzw. der Zusatzgeräte zu einer Einheit zu erzielen, ist eine Halteeinrichtung an dem Fibroskopteil oder an dem betreffenden Zusatzgerät vorgesehen. Vorzugsweise können der Fibroskopteil und das bzw. die Zusatzgeräte mittels der Halteeinrichtung wahlweise aneinander fixiert oder derart voneinander freigegeben werden, daß eine Relativbewegung zwischen Fibroskopteil und Zusatzgerät möglich ist.

Beispielsweise kann als Halteeinrichtung an dem bezüglich des bedienenden Arztes distalen Ende des Endoskops eine Schlinge - beispielsweise aus Nylon - vorgesehen sein, in der ein Zusatzgerät gehalten oder geführt wird. Es ist möglich, die Schlinge aus einer zentralen oder einer seitlichen Öffnung am distalen Ende des Fibroskopteils herausstehen zu lassen.

Insbesondere kann an dem Fibroskopteil ein Arbeitskanal von beispielsweise 1 mm vorgesehen sein, in dem eine Faßzange von beispielsweise 0,8 mm Durchmesser geführt ist, welche an einer geschlossenen Schlinge von ca. 60 mm Gesamtlänge angreift . Wenn diese Schlinge aus dem Arbeitskanal herausragt und dort ein Zusatzgerät umgreift, kann durch Zurückziehen oder Einschieben der Faßzange das betreffende Zusatzgerät am distalen Ende des Fibroskopteils über die Schlinge fixiert bzw. für eine Relativbewegung freigegeben werden.

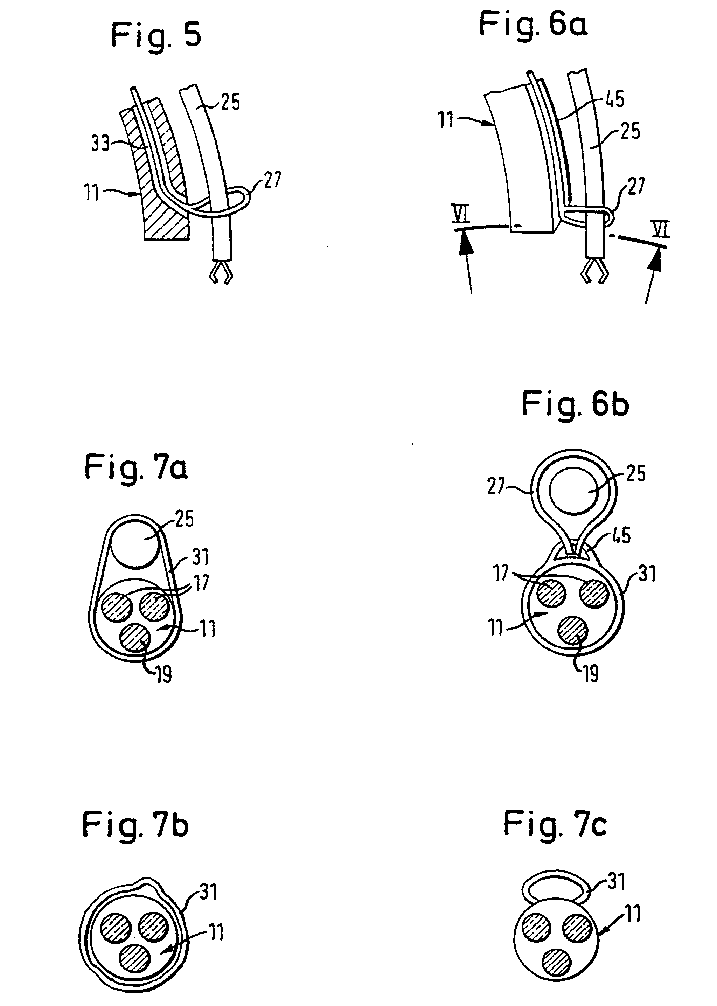

Alternativ hierzu ist es möglich, innerhalb eines Arbeitskanals des Fibroskopteils eine vergleichsweise lange Schlinge vorzusehen, die am distalen Ende des Endoskops ein Zusatzgerät umgreift und am proximalen Ende des Endoskops von dem Arzt bedient, insbesondere gezogen oder freigegeben wird (vgl. Fig. 5, 6a, 6b). Auf diese Weise kann auf die vorstehend erläuterte Faßzange innerhalb des Arbeitskanals verzichtet werden.

Außerdem ist es unter entsprechender Anwendung der vorstehend erläuterten Prinzipien möglich, anstelle einer Schlinge eine durch einen Arbeitskanal geführte Leine zu verwenden, die mit dem betreffenden Zusatzgerät fest verbunden ist. Dadurch kann verhindert werden, daß das Zusatzgerät bei freigegebener Schlinge unbeabsichtigt die Umgreifung durch die Schlinge verläßt.

Bei der Ausbildung des erfindungsgemäßen Endoskops mit voneinander separaten Fibroskopteil und Zusatzgerät ist es weiterhin bevorzugt, wenn entlang des Einführabschnitts des Fibroskopteils eine oder mehrere Befestigungsschlaufen als Halteeinrichtung vorgesehen sind, in denen jeweils ein oder mehrere Zusatzgeräte durch loses Umgreifen geführt sind.

Alternativ oder zusätzlich zu der Verwendung einer Schlinge können zur Aufnahme des Zusatzgeräts ein den Fibroskopteil vollständig oder teilweise umgebender Mantelschlauch (vgl. Fig. 7a bis 7c) oder eine an dem Fibroskopteil seitlich angeformte oder befestigte Seitenhülle (vgl. Fig. 6a, 6b) vorgesehen sein. Der Mantelschlauch bzw. die Seitenhülle kann aus Kunststoff und/oder elastisch ausgebildet sein. Außerdem kann der Mantelschlauch bzw. die Seitenhülle entlang des gesamten Einführabschnitts des Endoskops oder entlang lediglich eines oder mehrerer Teile hiervon vorgesehen sein.

Sofern der Mantelschlauch bzw. die Seitenhülle sich lediglich entlang eines Teilabschnitts des Endoskops erstreckt, kann der Mantelschlauch bzw. die Seitenhülle verschiebbar bezüglich des Fibroskopteils ausgebildet sein. Dadurch kann beispielsweise bei der Anwendung des Endoskops als Pharingoösophagogastroskop gewährleistet werden, daß der Mantelschlauch bzw. die Seitenhülle stets innerhalb des Nasengangs und sich bis zum Rachenraum erstreckend angeordnet ist, und zwar unabhängig von der Eindringtiefe des Fibroskopteils.

Falls zum Beispiel beim nasalen Einführen des Pharingoösophagogastroskops der verschiebbare Mantelschlauch zunächst am Vorderende, also am distalen Ende des Fibroskopteils angeordnet ist, kann der Mantelschlauch ab einer bestimmten Eindringtiefe des Fibroskopteils seine Position im Nasengang beibehalten, während der Fibroskopteil noch weiter eingeführt wird, beispielsweise zur Beobachtung der Speiseröhre. Auch wenn nachfolgend zur Beobachtung des Rachens der Fibroskopteil entsprechend zurückgezogen wird, behält der Mantelschlauch seine Position bei und ist somit wieder entlang des distalen Endes des Fibroskopteils angeordnet. Erst wenn der Fibroskopteil vollständig aus dem Nasengang herausgezogen wird, wird gleichzeitig der Mantelschlauch vom distalen Ende des Fibroskopteils mitgenommen.

Der besondere Vorteil dieser Ausführungsform liegt darin, daß in jedem der genannten Zustände ein Zusatzgerät problemlos und schmerzfrei entlang des Fibroskopteils durch den Mantelschlauch hindurch über den Nasengang in den Rachenraum eingeführt werden kann. Der Mantelschlauch verhindert nämlich eine Verletzung des Nasengangs und des Rachenraums durch das Zusatzgerät, und das distale Ende des Zusatzgeräts kann sich nicht aufgrund der zu überwindenden Krümmung unbeabsichtigt von dem Fibroskopteil lösen.

Die erläuterte Verschiebbarkeit des Mantelschlauchs kann realisiert werden, indem der Mantelschlauch bezüglich sowohl des Fibroskopteils als auch des Zusatzgeräts verschoben werden kann und als loser Schlauchabschnitt den Fibroskopteil und gegebenenfalls das Zusatzgerät umhüllt. Die Seitenhülle kann beispielsweise über eine einfache oder doppelte Schienen-Nut-Verbindung mit dem Fibroskopteil verschiebbar verbunden sein. Überdies kann am proximalen Ende des Mantelschlauchs bzw. der Seitenhülle ein Anschlagelement, beispielsweise eine ringförmige Verbreiterung vorgesehen sein, um ein vollständiges Verschwinden im Nasengang zu verhindern. Außerdem kann das Endoskop eine Arretiereinrichtung, beispielsweise eine Schraube aufweisen, über die der Mantelschlauch bzw. die Seitenhülle temporär bezüglich des Fibroskopteils fixiert werden kann.

Als Halteeinrichtung zum Halten oder Führen von Fibroskopteil und Zusatzgerät können ferner wenigstens eine Nut und eine hierzu entsprechende Schiene vorgesehen sein, die sich jeweils entlang des gesamten Einführabschnittes des Endoskops oder entlang einer oder mehrerer Teile hiervon erstrecken (vgl. Fig. 8, 9). Anstelle der Nut können auch eine oder mehrere Halteklammern an dem Fibroskopteil oder an dem Zusatzgerät vorgesehen sein (vgl. Fig. 12, 13).

Die an dem Fibroskopteil und dem Zusatzgerät vorgesehene Halteeinrichtung ist erfindungsgemäß durch einen oder mehrere Permanentmagnete, beispielsweise aus einer Eisen- oder Nickellegierung, gebildet, die mit wenigstens einem Gegenelement aus einem permanentmagnetischen oder magnetischen Material, beispielsweise Stahl, Aluminium oder Titan, zusammenwirken.

In einer besonders einfachen Ausführungsform kann dieses Gegenelement durch das Zusatzgerät bzw. das Fibroskopteil selbst gebildet sein. Außerdem ist es möglich, die Permanentmagnete oder die Gegenelemente an mehreren Abschnitten des Fibroskopteils bzw. des Zusatzgeräts vorzusehen. Um einen direkten Kontakt der verwendeten magnetischen Materialien zu vermeiden, und um sowohl die zwischen Zusatzgerät und Fibroskopteil herrschende Haftreibung als auch die Gleitreibung zu vermindern, können der Permanentmagnet, das Gegenelement oder beide eine Abdeckung aus Kunststoff besitzen.

Ferner können als Halteeinrichtung an dem Fibroskopteil einerseits und an dem Zusatzgerät andererseits ein Mitnehmerelement und eine Einhakeinrichtung vorgesehen sind, oder umgekehrt. Dadurch ist es möglich, das eingehakte Zusatzgerät mittels des Fibroskopteils in die Körperöffnung mit einzuführen, insbesondere zu ziehen. Danach kann das Zusatzgerät ausgeklinkt werden, um von der Lage des Fibroskopteils unabhängige Bewegungen und Untersuchungen durchzuführen.

Vorzugsweise sind das Mitnehmerelement und die Einhakeinrichtung jeweils am distalen Eride des Fibroskopteils bzw. des Zusatzgeräts angeordnet. Die Einhakeinrichtung kann beispielsweise durch einen seitlich abstehenden Hinterschneidungsansatz und das Mitnehmerelement durch einen seitlich abstehenden, in den Hinterschneidungsansatz gegebenenfalls eingreifenden Knopfansatz gebildet sein. Falls das Mitnehmerelement und die Einhakeinrichtung zum proximalen Ende des Endoskops hin abgeflacht ausgebildet sind, kann das Herausziehen des Fibroskopteils bzw. des Zusatzgeräts aus der Körperöffnung besonders leicht und sicher erfolgen.

Selbstverständlich ist es auch möglich, an einem einzigen Endoskop mehrere der genannten Halteeinrichtungen zu kombinieren.

Jede der vorstehend erläuterten Ausführungsformen des erfindungsgemäßen Endoskops kann wenigstens einen Arbeitskanal aufweisen, in den ein Hilfsmittel oder ein Zusatzgerät eingeführt werden kann oder der zur Durchführung von Spülungen oder zum Absaugen von Körperflüssigkeiten dienen kann.

Als das im Zusammenhang mit der Erfindung genannte Zusatzgerät, können beispielsweise eine Biopsie-Zange, eine Aspirator-/Injektorsonde, eine pH-Meßsonde, eine Manometrie-Sonde beispielsweise zur Untersuchung der Speiseröhren-Peristaltik, eine Bilitec-Meßsonde zur Messung des Bilirubin-Gehalts, eine Laser-Sonde oder andere chirurgische Instrumente für therapeutische Maßnahmen vorgesehen sein. Außerdem können aufgrund der optimierten Raumausnutzung des erfindungsgemäßen Endoskops auch mehrere Zusatzgeräte vorgesehen sein, beispielsweise eine Biopsie-Zange und eine pH-Meßsonde.

Für den bzw. die Licht-/Bildübertragungskanäle kann selbstverständlich die bekannte Aufteilung in einen Lichtübertragungskanal einerseits und einen Bildübertragungskanal andererseits vorgesehen sein. Beispielsweise können eine oder mehrere Lichtleiter einen Lichtübertragungskanal bilden. Der Bildübertragungskanal kann ebenfalls durch Lichtleiter und zugehörige Optiken gebildet sein, oder er kann einen opto-elektronischen Bildwandler am distalen Ende des Endoskops sowie entsprechende elektrische Versorgungs- und Übertragungsleitungen aufweisen.

Ferner kann das erfindungsgemäße Endoskop ein oder mehrere Bowdenzugsysteme zur aktiven seitlichen Ausrichtung besitzen. In diesem Fall kann die Schwenkrichtung mit der Richtung der größeren oder der geringeren Querschnitts-Abmessung des Endoskops zusammenfallen. Bei einem nasal einzuführenden Endoskop ist es hinsichtlich der für das Passieren des Rachenraums erforderlichen Richtungsänderung von Vorteil, wenn das Endoskop zumindest in der Richtung der größeren Querschnitts-Abmessung des Endoskops aktiv verschwenkbar ist.

Das erfindungsgemäße Endoskop mit seinen besonderen Vorteilen kann in vielen Bereichen der Endoskopie zur Anwendung gelangen, beispielsweise in der Bronchoskopie. Eine vorteilhafte Anwendungsmöglichkeit besteht auch in der Spülung von Körperhöhlen, beispielsweise von eustachischer Röhre oder Kieferhöhle. Außerdem findet die Erfindung Anwendung in der Chirurgie, insbesondere für die Durchführung steriler Arbeiten mit Hilfe von Zusatzgeräten unter gleichzeitiger optischer Beobachtung.

Weitere bevorzugte Ausführungsformen des Endoskops gehen aus den Unteransprüchen hervor. Die Erfindung wird nachfolgend beispielhaft unter Bezugnahme auf die Zeichnungen beschrieben; in diesen zeigen:
- Fig. 1, 8, 10, 12, 14 und 16: schematische Seitenansichten jeweils eines Endoskops,

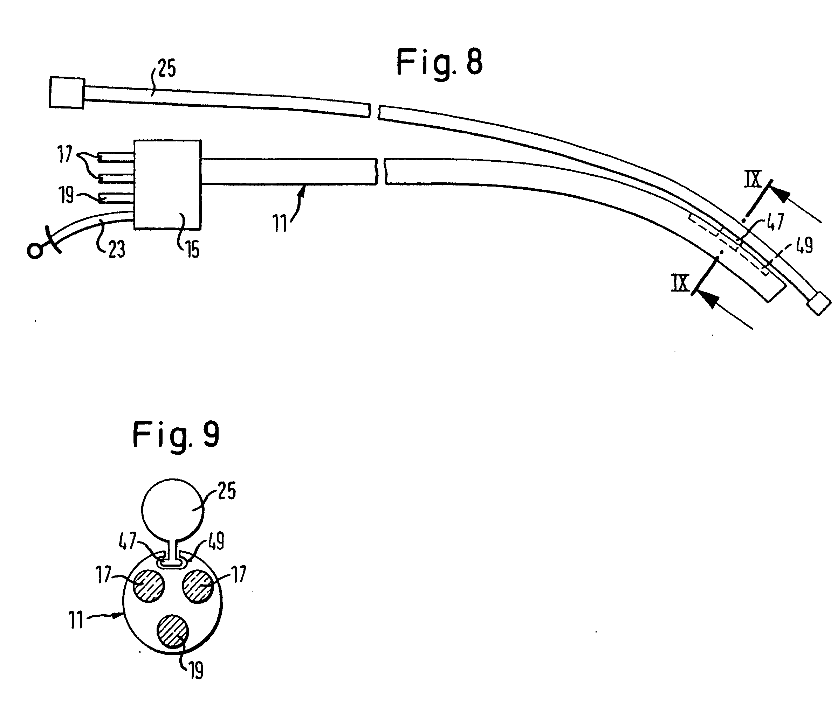

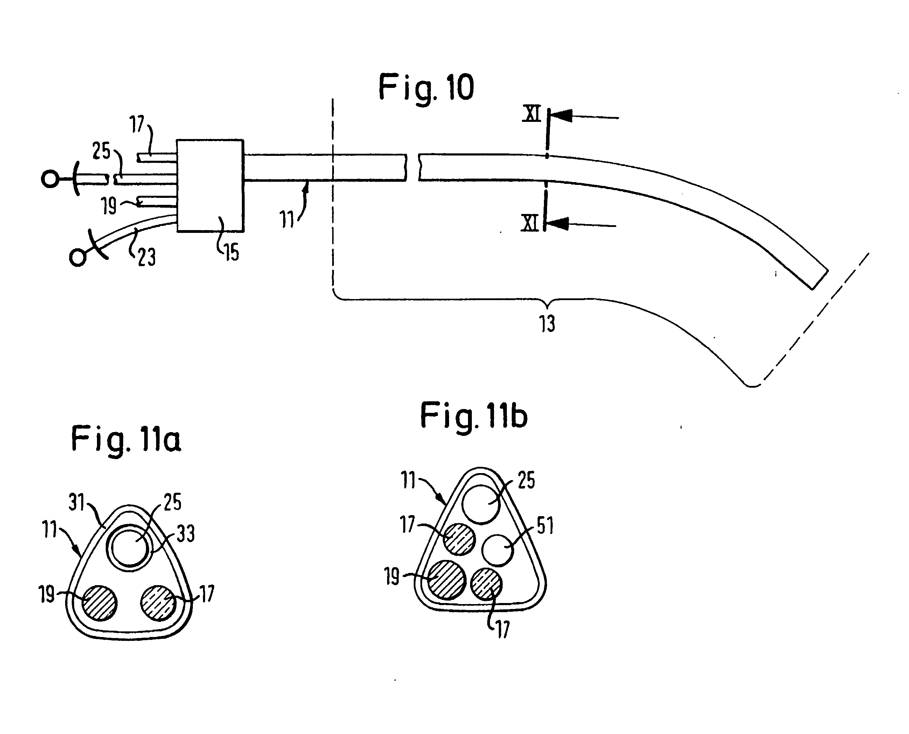

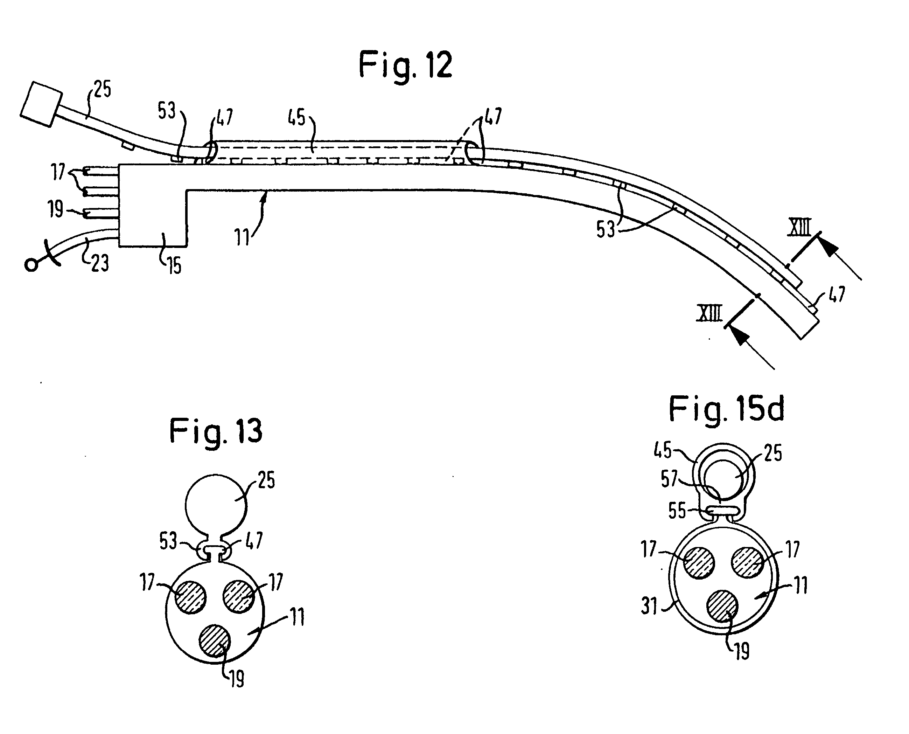

- Fig. 2, 6b, 9, 11a, 11b und 13: jeweils einen Schnitt entlang der Ebene II-II gemäß Fig. 1, der Ebene VI-VI gemäß Fig. 6a, der Ebene IX-IX gemäß Fig. 8, der Ebene XI-XI gemäß Fig. 10, der Ebene XI-XI gemäß Fig. 10, bzw. entlang der Ebene XIII-XIII gemäß Fig. 12,
- Fig. 3 und 3b: Detailansichten des Bereichs III gemäß Fig. 1,

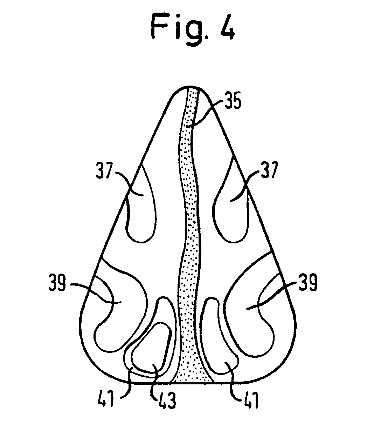

- Fig. 4: einen schematischen Frontalschnitt durch eine menschliche Nase,
- Fig. 5 und 6a: den Fig. 3a und 3b entsprechende Detailansichten jeweils eines weiteren Endoskops,
- Fig. 7a bis 7c: der Fig. 2 entsprechende Schnittansichten weiterer Endoskope,

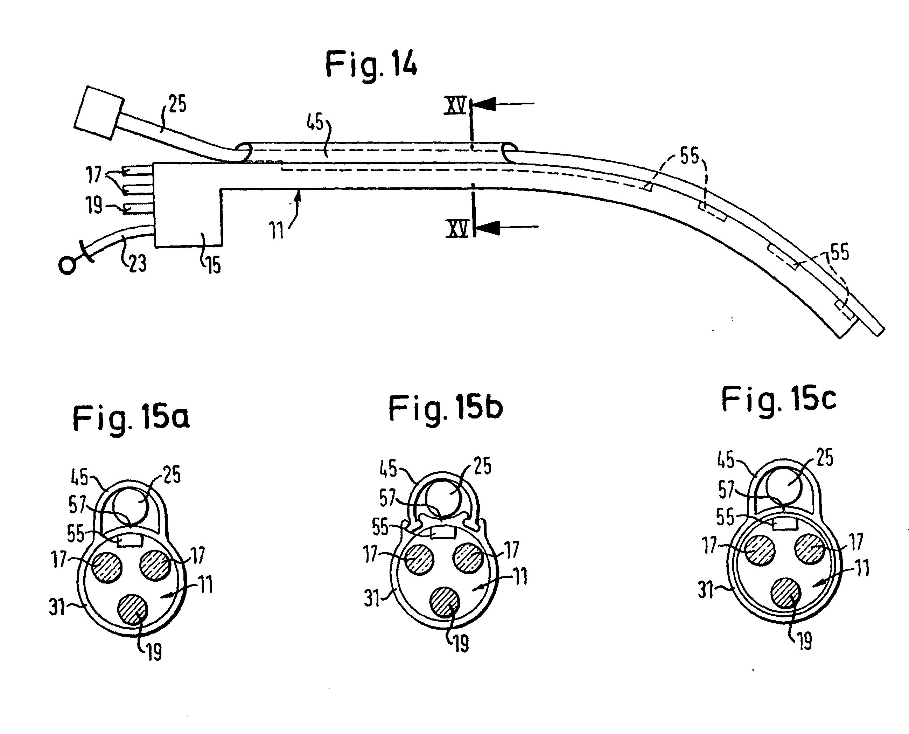

- Fig. 15a, 15b, 15c und 15d: jeweils einen Schnitt entlang der Ebene XV-XV gemäß Fig. 14 für verschiedene Ausführungsformen,

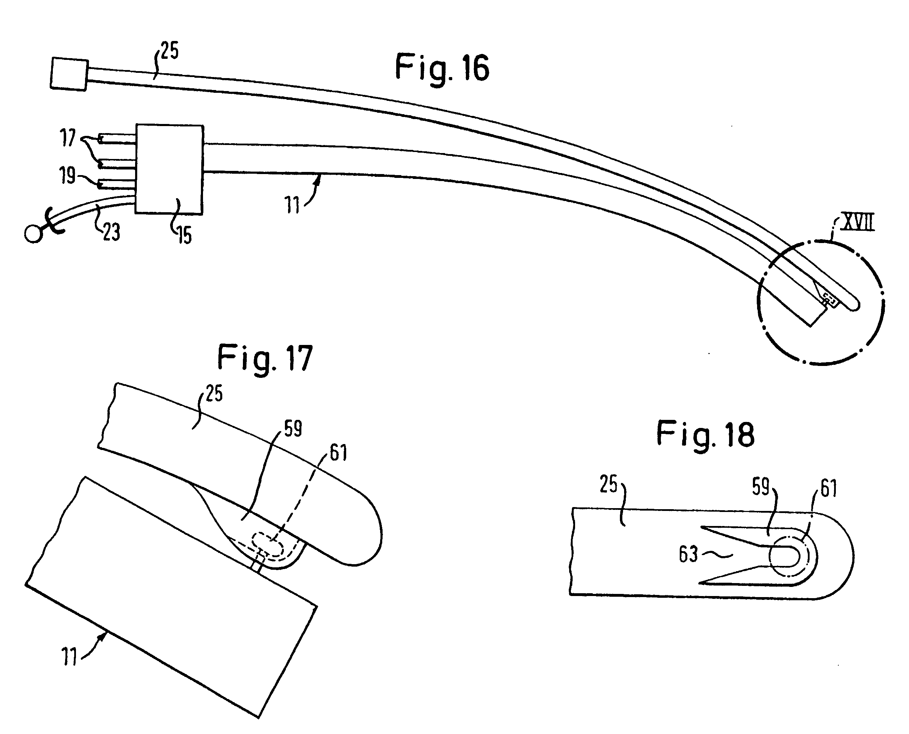

- Fig. 17: eine Detailansicht des Bereichs XVII gemäß Fig. 16, und
- Fig. 18: die Unterseite des distalen Endes des Zusatzgeräts gemäß Fig. 16 in einer Detailansicht.

Lediglich die Fig. 14, 15a, 15b, 15c und 15d zeigen Ausführungsformen der Erfindung.

Fig. 1 zeigt eine Seitenansicht eines Pharingoösophagogastroskops. Dieses weist ein Fibroskop 11 auf mit einem länglichen Einführabschnitt 13 und einem proximalen Bedienungsteil 15. An dem Bedienungsteil 15 sind die offenen Enden zweier Lichtübertragungskanäle 17 und eines optischen Bildübertragungskanals 19 sowie die bedienerseitigen Enden einer Faßzange 21 und eines Bowdenzugs 23 gezeigt, deren jeweiliger Verlauf innerhalb des Einführabschnitts 13 des Fibroskops 11 in Fig. 1 nicht dargestellt ist.

Ferner besitzt das Endoskop gemäß Fig. 1 ein Zusatzgerät 25 in Form einer länglichen Biopsie-Zange. Dieses Zusatzgerät 25 ist am distalen Ende des Endoskops über eine Schlinge 27 und nahe des Bedienungsteils 15 mittels einer Befestigungsschlaufe 29 mit dem Fibroskop 11 verbunden.

Fig. 2 zeigt in einem Schnitt durch den Einführabschnitt 13 des Endoskops gemäß Fig. 1, daß das Fibroskop 11 einen flexiblen Mantelschlauch 31 als Außenhülle besitzt sowie einen zentralen Arbeitskanal 33, in dem die Faßzange 21 geführt ist. Der Bowdenzug 23 ist hier nicht dargestellt.

Die Fig. 3a und 3b zeigen jeweils Detailansichten des Bereichs III gemäß Fig. 1, also des distalen Endes des Endoskops. Die im Arbeitskanal 33 geführte Faßzange 21 hält die Schlinge 27. Diese ragt zur Ermöglichung einer Relativbewegung von Fibroskop 11 und Biopsie-Zange 25 aus dem Fibroskop 11 heraus und umgreift die Biopsie-Zange 25 lose (Fig. 3a). Durch Zurückziehen der Faßzange 21 relativ zu dem Fibroskop 11 wird die Schlinge 27 tiefer in den Arbeitskanal 33 gezogen, so daß die freie Länge der Schlinge 27 sich verringert und die Biopsie-Zange 25 am distalen Ende des Fibroskops 11 fixiert wird (Fig. 3b). Anstelle der Faßzange 21 kann natürlich auch eine andere Art der Schlingenhalterung vorgesehen sein.

Das durch das Fibroskop 11 und das Zusatzgerät 25 gebildete Endoskop gemäß Fig. 1 bis 3b kann einem Patienten über einen Nasengang in den Rachen und nachfolgend in die Speiseröhre und den Magen eingeführt werden. Diese Anwendungsweise wird nachfolgend anhand der Fig. 4 erläutert. Diese zeigt einen Frontalschnitt einer Nase mit einer Nasenscheidewand 35, zwei mittleren Muscheln 37 und zwei unteren Muscheln 39. Die unteren Nasenmuscheln 39 und die Nasenscheidewand 35 begrenzen jeweils einen unteren Nasengang 41. In einem derartigen unteren Nasengang 41, vorzugsweise in den jeweils größeren, kann ein Endoskop eingeführt werden.

Aufgrund des letztlich länglichen Querschnitts der Nasengänge 41 kann ein Endoskop vergleichsweise großen Querschnitts 43 eingeführt werden, wenn dieser Querschnitt 43 - wie in Fig. 4 gezeigt - eine nicht kreisrunde, sondern der betreffenden Körperöffnung 41 angepaßte Form besitzt. Mit anderen Worten können mit dem derartig geformten Endoskop aufgrund der verbesserten Flächenausnutzung mehr oder größere Zusatzgeräte bzw. Licht-/ Bildübertragungskanäle durch den Nasengang eingeführt werden als bei einem herkömmlichen Fibroskop runden Querschnitts.

Dementsprechend ist der Gesamtquerschnitt des Endoskops gemäß Fig. 1 nicht kreisrund, sondern - wie für die Einheit aus Fibroskop 11 und Zusatzgerät 25 aus Fig. 2 ersichtlich - dem in Fig. 4 gezeigten Querschnitt 43 nachempfunden. Der angepaßte Querschnitt des Endoskops verleiht diesem auch eine Stabilität bezüglich einer unerwünschten Drehung um seine Längsachse. Dies ist insbesondere bei einer aktiven Krümmung und Ausrichtung des Endoskops mittels eines Bowdenzugs von Vorteil.

Wird das Endoskop gemäß Fig. 1 also - wie für den Querschnitt 43 in Fig. 4 gezeigt - über den Nasengang 41 eingeführt, so kann das flexible Zusatzgerät 25 über die Schlinge 27 von dem distalen Ende des Fibroskops 11 geführt werden, und zwar durch entsprechende Betätigung des Bowdenzugs 23. Gleichzeitig kann auf bekannte Weise die betreffende Körperregion über die Lichtübertragungskanäle 17 beleuchtet und über den Bildübertragungskanal 19 und entsprechende Optik und Videotechnik beobachtet werden.

Zu dem Endoskop gemäß Fig. 1 ist anzumerken, daß zwar auch über die in dem zentralen Arbeitskanal 33 geführte Faßzange 21 eine Probeentnahme möglich ist. Allerdings besitzt der Arbeitskanal 33 des Fibroskops 11 typischerweise einen Innendurchmesser von lediglich 1 mm. Die Faßzange 21 von dementsprechend weniger als 1 mm Außendurchmesser kann lediglich Schleimhautgewebe entnehmen. Dagegen vermag die Biopsie-Zange 25 des erfindungsgemäßen Endoskops aufgrund ihres größeren Durchmessers, Proben auch aus tieferen Gewebelagen zu entnehmen.

Anhand der Fig. 5 bis 18 werden nachfolgend weitere Ausführungsbeispiele von Endoskopen beschrieben, wobei gleiche oder gleichartige Elemente wie in den Fig. 1 bis 3b jeweils durch dieselben Bezugszeichen gekennzeichnet sind.

Das Ausführungsbeispiel gemäß Fig. 5 unterscheidet sich von dem Endoskop gemäß Fig. 1 dadurch, daß die Schlinge 27 nicht von einer eigenen Faßzange 21 gehalten wird, sondern letztlich einsträngig durch den Arbeitskanal 33 bis zu einem nicht gezeigten Bedienungsteil geführt ist. Der Arbeitskanal 33 mündet hier nicht an der distalen Stirnseite, sondern an einem Seitenabschnitt des distalen Endes des Fibroskops 11.

Auch bei dem Ausführungsbeispiel gemäß Fig. 6a ist eine einzige Schlinge 27 ohne Faßzange vorgesehen. Die Schlinge 27 ist - im Gegensatz zu Fig. 5 - nicht in einem zentralen Arbeitskanal, sondern innerhalb einer an dem Fibroskop 11 angeformten Seitenhülle 45 geführt. Dieses Prinzip ist auch in der frontalen Schnittansicht gemäß Fig. 6b illustriert. Die angeschrägte Öffnung der Seitenhülle 45 am distalen Ende des Fibroskops 11 kann mit einem Endstück ("dummy") verschlossen werden, falls das Zusatzgerät 25 und somit die Schlinge 27 nicht erforderlich sind.

Fig. 7a zeigt ein weiteres Endoskop in einer Querschnittsansicht seines Einführabschnittes. Auch hier sind ein Fibroskop 11 und ein Zusatzgerät 25 als im wesentlichen separate Bauteile vorgesehen, die über einen elastischen gemeinsamen Mantelschlauch 31 miteinander verbunden sind. Wie aus Fig. 7b hervorgeht, zieht sich dieser Mantelschlauch 31 bei Entnahme des Zusatzgeräts 25 zusammen, um als Außenhülle lediglich des Fibroskops 11 zu dienen.

In Fig. 7c ist gezeigt, daß der Mantelschlauch 31 - ähnlich der Seitenhülle 45 gemäß Fig. 6a und 6b - auch lediglich an einem Teil des Umfangs des Fibroskops 11 vorgesehen sein kann, um dort gegebenenfalls ein Zusatzgerät aufzunehmen.

Bei dem Endoskop gemäß Fig. 8 sind ein Fibroskop 11 und ein Zusatzgerät 25 in Form einer Aspirator-/Injektorsonde als voneinander unabhängige Einheiten ausgebildet, die lediglich durch eine gemeinsame Halteeinrichtung miteinander verbunden sind. Wie die Schnittansicht gemäß Fig. 9 zeigt, ist diese Halteeinrichtung durch einen an dem Zusatzgerät 25 angeformten Führungsansatz in Form einer Schiene 47 sowie seitens des Fibroskops 11 durch eine entsprechende Nut 49 gebildet. Die Nut 49 erstreckt sich entlang der Längsrichtung des Fibroskops 11 um eine deutlich größere Länge als die Länge der Schiene 47, so daß eine Längsverschiebung von Fibroskop 11 und Zusatzgerät 25 relativ zueinander lediglich innerhalb vorbestimmter Grenzen möglich ist.

Bei diesem Endoskop kann zur zusätzlichen Stabilisierung von Fibroskop 11 und Zusatzgerät 25 eine Stabilisierungseinrichtung außerhalb des Körpers, insbesondere nahe des Bedienungsteils 15 vorgesehen sein. Diese Stabilisierungseinrichtung kann ebenfalls durch ein Nut-Schiene-System gebildet sein und ist vorzugsweise an das Fibroskop 11 und/oder das Zusatzgerät 25 anklippbar.

Weiterhin zeigt Fig. 10 ein Endoskop, das im Unterschied zu den Ausführungsbeispielen gemäß Fig. 1 bis 9 als eine einzige geschlossene Einheit ausgebildet ist, und dessen Einführäbschnitt 13 erfindungsgemäß einen nicht kreisrunden Querschnitt aufweist. Für dieses Endoskop ist ein Zusatzgerät 25 - beispielsweise eine Biopsie-Zange - vorgesehen, welches im Vergleich zu Zusatzgeräten entsprechender herkömmlicher Endoskope einen deutlich größeren Querschnittdurchmesser aufweisen kann. Dies ist möglich, da der nicht rotationssymmetrische Querschnitt des Endoskops eine flexiblere Anordnung des Zusatzgeräts 25, der Licht-/Bildübertragungskanäle 17, 19 und des Bowdenzugs 23 innerhalb des Einführabschnitts 13 erlaubt, wobei das Zusatzgerät 25 nicht unbedingt an zentraler Stelle angeordnet ist.

Das Endoskop gemäß Fig. 10 kann beispielsweise den in Fig. 11a gezeigten Querschnitt aufweisen. Hier bilden ein Lichtübertragungskanal 17, ein Bildübertragungskanal 19 und ein innerhalb eines Arbeitskanals 33 geführtes Zusatzgerät 25 eine von einem Mantelschlauch 31 umgebene Einheit, die einem gleichschenkligen Dreieck oder Trapez mit abgerundeten Ecken gleicht.

Der für das Endoskop gemäß Fig. 10 ebenfalls mögliche Querschnitt gemäß Fig. 11b besitzt eine vergleichbare, im wesentlichen dreieckige Form. Hier ist das Zusatzgerät 25 fest in den Endoskop integriert, welches gleichwohl - wie in Fig. 10 ersichtlich - verformt und seitlich ausgerichtet werden kann. Außerdem sind bei der speziellen Ausführungsform gemäß Fig. 11b zwei Lichtübertragungskanäle 17, ein Bildübertragungskanal 19 sowie ein eigener Spülkanal 51 vorgesehen.

Fig. 12 zeigt wiederum eine Ausführungform mit einem von dem Fibroskopteil 11 unabhängigen Zusatzgerät 25. Das Zusatzgerät 25 besitzt, ähnlich der Schienen-Nut-Verbindung gemäß Fig. 8 und 9, über seine Länge in gleichmäßigen Abständen verteilt mehrere Halteklammern 53, die jeweils seitlich von dem Zusatzgerät 25 abstehen und eine entlang des Fibroskopteils 11 gebildete Schiene 47 umgreifen. Wie der Querschnittsansicht gemäß Fig. 13 zu entnehmen ist, besitzen die Halteklammern 53 einen C-förmigen und die Schiene 47 einen T-förmigen Querschnitt.

Entlang eines proximalen Abschnitts des Fibroskopteils 11 ist an diesem eine Seitenhülle 45 angeformt, die das Aufsetzen des Zusatzgeräts 25 auf die Schiene 47 des Fibroskopteils 11 erleichtert und das Einführen des Fibroskopteils 11 mit aufgesetztem Zusatzgerät 25 in die betreffende Körperöffnung, insbesondere den Nasengang unterstützt. Das derartig aufgesetzte Zusatzgerät 25 kann beliebig und ohne Begrenzung entlang des Fibroskopteils 11 verfahren werden. Insbesondere kann das Zusatzgerät 25 bei bereits in die Körperöffnung eingeführtem Fibroskopteil 11 nachgeschoben werden, wobei der Fibroskopteil 11 für die erforderliche Führung sorgt.

Bei dem Endoskop gemäß Fig. 14 ist als Zusatzgerät 25 eine Biopsie-Zange aus einem magnetisierbaren Metall vorgesehen. Wie aus der Querschnittsansicht gemäß Fig. 15a ersichtlich, ist in den Fibroskopteil 11 ein Permanentmagnet 55 integriert, der das Zusatzgerät 25 magnetisch anzuziehen vermag. Aufgrund dieses magnetischen Zusammenwirkens wird das Zusatzgerät 25 entlang des Fibroskopteils 11 geführt.

Entlang eines proximalen Teils des Fibroskopteils 11 ist das Zusatzgerät 25 zur zusätzlichen Führung und Stabilisierung durch eine Seitenhülle 45 geführt. Entlang eines distalen Teils des Fibroskopteils 11 ist der Permanentmagnet 55 lediglich abschnittsweise vorgesehen, so daß an den betreffenden Abschnitten ein beabsichtigtes Lösen des distalen Endes des Zusatzgeräts 25 von dem Fibroskopteil 11 möglich ist.

Indem der den Fibroskopteil 11 umgebende und in die Seitenhülle 45 übergehende Mantelschlauch 31 auch als Abdeckung 57 des Permanentmagneten 55 dient, wird die Reibung zwischen dem Fibroskopteil 11 und dem Zusatzgerät 25 vermindert und die relative Beweglichkeit erhöht.

Fig. 15b zeigt die Querschnittsansicht einer modifizierten Ausführungsform, bei der die Seitenhülle 45 über eine doppelte Schienen-Nut-Verbindung an dem Mantelschlauch 31 befestigt ist und somit entlang des gesamten Fibroskopteils 11 verschoben werden kann. Dadurch kann die Seitenhülle 45 unabhängig von der Einführtiefe des Fibroskopteils 11 stets im Nasengang 41 (Fig. 4) des Patienten belassen werden, um das schmerz- und verletzungsfreie Nachführen des Zusatzgeräts 25 zu erleichtem. Die im Querschnitt T-förmigen Schienen der Seitenhülle 45 erstrecken sich über die gesamte Länge der Seitenhülle 45. Die entsprechenden Nuten am Fibroskopteil 11 verlaufen entlang des gesamten Einführabschnitts 13 bis kurz vor das distale Ende des Fibroskopteils 11.

Fig. 15c zeigt eine weitere Ausführungsform, bei der die Seitenhülle 45 an dem Mantelschlauch 31 angeformt ist. Im Unterschied zu der Ausführungsform gemäß Fig. 15a ist die Einheit aus Seitenhülle 45 und Mantelschlauch 31 hier lose über den Fibroskopteil 11 übergezogen, so daß diese Einheit relativ sowohl zum Fibroskopteil 11 als auch zum Zusatzgerät verschoben werden kann. Ein nicht gezeigter Stopper am distalen Ende des Fibroskopteils 11 kann ein unbeabsichtigtes Lösen vom Fibroskopteil 11 verhindern.

Fig. 15d zeigt ein der Ausführungsform gemäß Fig. 15b entsprechendes weiteres Beispiel einer Schienen-Nut-Verbindung eines Mantelschlauchs 31 mit einer separaten Seitenhülle 45, die lediglich entlang eines Teilabschnitts des Fibroskopteils 11 vorgesehen ist und relativ hierzu verschoben werden kann. Bei diesem Beispiel ist eine Schiene seitens des Fibroskopteils 11 vorgesehen, während eine entsprechende Nut an der Seitenhülle 45 angeordnet ist. Diese umgekehrte Anordnung von Schiene und Nut kann in manchen Anwendungen eine Verletzungsgefahr verringern.

Bei dem Ausführungsbeispiel gemäß Fig. 15d ist außerdem die Schiene durch den im Querschnitt abgerundeten Permanentmagneten 55 selbst gebildet. Dadurch wird innerhalb des Fibroskopteils 11 eine Volumenverringerung erzielt, und das Zusatzgerät 25 kann zugunsten einer höheren magnetischen Anziehung zumindest außerhalb der Seitenhülle 45 unmittelbar an dem Permanentmagneten 55 anliegen.

Schließlich ist es bei dem Ausführungsbeispiel gemäß Fig. 15d alternativ möglich, daß die Seitenhülle 45 die einzige Schiene 55 klammerartig umgreift ohne Abdeckungssteg 57, so daß das Zusatzgerät 25 auch innerhalb der Seitenhülle 45 direkt auf der Schiene 55 aufliegen kann.

Fig. 16 und 17 zeigen ein Endoskop mit einer Manometrie-Sonde als Zusatzgerät 25. Dieses ist mit seinem distalen Ende an dem distalen Ende des Fibroskopteils 11 eingehakt. Hierfür ist an dem Zusatzgerät 25 eine Einhakeinrichtung 59 in Form eines seitlich abstehenden Hinterschneidungsansatzes vorgesehen, die ein Mitnehmerelement 61 des Fibroskopteils 11 hintergreift und teilweise umgreift. Das Mitnehmerelement 61 ist durch einen von dem Fibroskopteil 11 seitlich abstehenden Knopfansatz gebildet, der entlang der Längsrichtung des Zusatzgeräts 25 in einen an dem Hinterschneidungsansatz 59 gebildeten Längsschlitz 63 eingeführt ist. Die Lage des derartig eingehakten Mitnehmerelements 61 geht aus Fig. 18 hervor, welche die Unterseite des Zusatzgeräts 25 in einer Detailansicht zeigt.

Das derartig eingehakte Zusatzgerät 25 kann gemeinsam mit dem Fibroskopteil 11 in die Körperöffnung eingeführt werden. Falls danach das Zusatzgerät 25 über den Fibroskopteil 11 hinausgeschoben wird, verläßt das Mitnehmerelement 61 die Einhakeinrichtung 59. Dadurch kann mit dem Zusatzgerät 25 unabhängig von dem Fibroskopteil 11 agiert werden, und daß Zusatzgerät 25 kann insbesondere unabhängig von dem Fibroskopteil 11 der Körperöffnung wieder entnommen werden.

### Bezugszeichenliste

- 11: Fibroskop
- 13: Einführabschnitt
- 15: Bedienungsteil
- 17: Lichtübertragungskanal
- 19: Bildübertragungskanal
- 21: Faßzange
- 23: Bowdenzug
- 25: Zusatzgerät
- 27: Schlinge
- 29: Befestigungsschlaufe
- 31: Mantelschlauch
- 33: Arbeitskanal
- 35: Nasenscheidewand
- 37: mittlere Muschel
- 39: untere Muschel
- 41: unterer Nasengang
- 43: Querschnitt
- 45: Seitenhülle
- 47: Schiene
- 49: Nut
- 51: Spülkanal
- 53: Halteklammer
- 55: Permanentmagnet
- 57: Abdeckung
- 59: Einhakeinrichtung
- 61: Mitnehmerelement
- 63: Längsschlitz

## Patentansprüche

1. Verformbares Endoskop, das einen oder mehrere Licht-/Bildübertragungskanäle (17, 19) und wenigstens ein Zusatzgerät (25) aufweist,
wobei der Licht-/Bildübertragungskanal bzw. die mehreren Licht-/Bildübertragungskanäle (17, 19) - insbesondere gemeinsam mit wenigstens einem Arbeitskanal (33) - einen geschlossenen Fibroskopteil (11) bilden,
wobei der Fibroskopteil (11) von dem Zusatzgerät (25) trennbar ist,
wobei eine Halteeinrichtung vorgesehen ist zum Halten und/oder Führen von Fibroskopteil (11) und Zusatzgerät (25) relativ zueinander,
wobei als Halteeinrichtung an dem Fibroskopteil (11) einerseits und an dem Zusatzgerät (25) andererseits wenigstens ein Permanentmagnet (55) bzw. wenigstens ein Gegenelement (25) aus permanentmagnetischem oder magnetischem Material vorgesehen sind, oder umgekehrt,
**dadurch gekennzeichnet,**
**daß** die Einheit aus Fibroskopteil (11) und Zusatzgerät (25) entlang eines in eine menschliche oder tierische Körperöffnung (41) einzuführenden Längsabschnitts (13) (Einfuhrabschnitt) einen nicht runden Querschnitt (43) aufweist, und
**daß** der Fibroskopteil (11) und das Zusatzgerät (25) entlang ihrer Längsrichtung relativ zueinander verschiebbar sind.

2. Endoskop nach Anspruch 1,
**dadurch gekennzeichnet,**
**daß** das Zusatzgerät (25) mittels der Halteeinrichtung wahlweise an dem Fibroskopteil (11) fixierbar oder zur Ermöglichung einer Relativbewegung entlang der Längsrichtung des Zusatzgeräts (25) und des Fibroskopteils (11) von dem Fibroskopteil (11) freigebbar ist.

3. Endoskop nach einem der vorhergehenden Ansprüche,
**dadurch gekennzeichnet,**
**daß** die Halteeinrichtung am distalen Ende des Fibroskopteils (11) zur Aufnahme des Zusatzgeräts (25) eine Schlinge (27) zum Umgreifen des Zusatzgeräts (25) aufweist, deren freie Länge vorzugsweise aktiv veränderbar ist.

4. Endoskop nach Anspruch 3,
**dadurch gekennzeichnet,**
**daß** die Schlinge (27) von einer Faßzange (21) gehalten wird, die in einem an dem Fibroskopteil (11) vorgesehenen Arbeitskanal (33, 45) geführt ist, oder
**daß** die Schlinge (27) sowohl am distalen als auch am proximalen Ende eines an dem Fibroskopteil (11) vorgesehenen Arbeitskanals (33, 45) aus diesem herausragt.

5. Endoskop nach einem der vorhergehenden Ansprüche,
**dadurch gekennzeichnet,**
**daß** als Halteeinrichtung an dem Fibroskopteil (11) einerseits und an dem Zusatzgerät (25) andererseits eine Nut (49) oder Halteklammer (53) bzw. eine entsprechende Schiene (47) oder entsprechende Schienensegmente vorgesehen sind, oder umgekehrt.

6. Endoskop nach einem der vorhergehenden Ansprüche,
**dadurch gekennzeichnet,**
**daß** das Gegenelement ein integraler Teil des Zusatzgeräts (25) bzw. des Fibroskopteils ist, und/oder
**daß** der Permanentmagnet (55) an mehreren Abschnitten des Fibroskopteils (11) bzw. des Zusatzgeräts vorgesehen ist, und/oder daß der Permanentmagnet (55) und/oder das Gegenelement eine Abdeckung (57) aus Kunststoff aufweisen.

7. Endoskop nach einem der vorhergehenden Ansprüche,
**dadurch gekennzeichnet,**
**daß** als Halteeinrichtung an dem Fibroskopteil (11) einerseits und an dem Zusatzgerät (25) andererseits ein Mitnehmerelement (61) und eine Einhakeinrichtung (59) vorgesehen sind, oder umgekehrt.

8. Endoskop nach Anspruch 7,
**dadurch gekennzeichnet,**
**daß** das Mitnehmerelement (61) und/oder die Einhakeinrichtung (59) jeweils am distalen Ende des Fibroskopteils (11) bzw. des Zusatzgeräts (25) vorgesehen sind, und/oder
**daß** das Mitnehmerelement (61) durch einen seitlich abstehenden Knopfansatz und die Einhakeinrichtung (59) durch einen seitlich abstehenden Hinterschneidungsansatz gebildet ist, und/oder
**daß** das Mitnehmerelement (61) und/oder die Einhakeinrichtung (59) zum proximalen Ende des Endoskops hin abgeflacht ausgebildet sind.

9. Endoskop nach einem der vorhergehenden Ansprüche,
**dadurch gekennzeichnet,**
**daß** entlang des Einführabschnitts (13) des Endoskops und von dessen distalem Ende beabstandet wenigstens eine Befestigungsschlaufe (29) für die Führung des Zusatzgeräts (25) vorgesehen ist.

10. Endoskop nach einem der vorhergehenden Ansprüche,
**dadurch gekennzeichnet,**
**daß** als Halteeinrichtung an dem Fibroskopteil (11) zur Aufnahme des Zusatzgeräts (25) ein Mantelschlauch (31) oder eine Seitenhülle (45) vorgesehen ist, der bzw. die sich entlang des gesamten Einführabschnitts oder entlang eines Teils des Einführabschnitts (13) des Endoskops erstreckt.

11. Endoskop nach Anspruch 10,
**dadurch gekennzeichnet**,
der Mantelschlauch (31) sowohl den Fibroskopteil (11) als auch das Zusatzgerät (25) umgibt und/oder bezüglich seines Durchmessers elastisch ausgebildet ist, und/oder
daß der Mantelschlauch (31) bzw. die Seitenhülle (45) bezüglich des Fibroskopteils (11) verschiebbar ausgebildet ist, insbesondere aufgrund einer Schienen-Nut-Verbindung (Fig. 15b).

12. Endoskop nach einem der vorhergehenden Ansprüche,
**dadurch gekennzeichnet,**
**daß** der Querschnitt (43) des Einführabschnitts (13) an die Körperöffnung (41) angepaßt ist, und/oder
**daß** das Endoskop als Pharingoösophagogastroskop ausgebildet ist zur Untersuchung von Rachen, Speiseröhre und/oder Magen, wobei der Querschnitt (43) des Einführabschnitts (13) an den Querschnitt eines menschlichen Nasengangs (41) angepaßt ist.

13. Endoskop nach einem der vorhergehenden Ansprüche,
**dadurch gekennzeichnet,**
**daß** die Querschnittsabmessung des Einführabschnitts (13) in einer Richtung größer, insbesondere zumindest um einen Faktor von 1,5 größer ist als in einer hierzu orthogonalen Richtung, und/oder
**daß** der Querschnitt (43) des Einführabschnitts (13) einem gleichschenkligen Dreieck oder einem spiegelsymmetrischen Trapez entspricht, jeweils mit abgerundeten Ecken und vorzugsweise mit einer Basislänge von höchstens ca. 3,5 mm.

14. Endoskop nach einem der vorhergehenden Ansprüche,
**dadurch gekennzeichnet,**
**daß** das Zusatzgerät (25) durch eine Biopsie-Zange, eine Aspirator-/Injektorsonde, eine pH-Meßsonde, ein Druckmeßgerät und/oder eine Bilitec-Meßsonde gebildet ist, und/oder
**daß** die maximale Querschnittsabmessung des Zusatzgeräts (25) höchstens ca. 3 mm, vorzugsweise höchstens ca. 2 mm beträgt.

15. Endoskop nach einem der vorhergehenden Ansprüche,
**dadurch gekennzeichnet,**
**daß** das Zusatzgerät (25) bezüglich des Zentrums des Querschnitts (43) des Einführabschnitts (13) seitlich beabstandet vorgesehen ist, und/oder
**daß** der Fibroskopteil (11) und das Zusatzgerät (25) entlang ihrer Längsrichtung um eine Länge von bis zu ca. 5 cm oder von bis zu ca. 35 cm relativ zueinander verschiebbar sind.

16. Endoskop nach einem der vorhergehenden Ansprüche,
**dadurch gekennzeichnet,**
**daß** das Endoskop einen Bowdenzug (23) aufweist, durch den das Endoskop in der Richtung seiner größeren Querschnitts-Abmessung aktiv verschwenkbar ist, und/oder
**daß** als Licht-/Bildübertragungskanäle wenigstens ein separater Lichtübertragungskanal (17) und wenigstens ein separater Bildübertragungskanal (19) vorgesehen sind.

## Claims

1. A deformable endoscope that has one or more light/image transmission passages (17, 19) and at least one additional instrument (25), wherein the light/transmission passage or the plurality of light/image transmission passages (17, 19) - in particular together with at least one work channel (33) - form a closed fibroscope part (11);
wherein the fiboroscope part (11) can be separated from the additional instrument (25);
wherein a holding device is provided for the holding/and or guiding of the fibroscope part (11) and the additional instrument (25) relative to one another;
wherein at least one permanent magnet (55) or at least one counter-element (25) made of permanently magnetic or magnetic material are provided as a holding device at the fibroscope part (11), on the one hand, and at the additional instrument (25) on the other hand, or vice versa,
**characterised**
**in that** the unit of endoscope (11) and additional instrument (25) has a non-round cross-section (43) along a longitudinal section (13) (insertion section) to be introduced into a human or animal body orifice (41); and
**in that** the fibroscope part (11) and the additional instrument (25) can be displaced relative to one another along their longitudinal direction.

2. An endoscope in accordance with claim 1, **characterised in that** the additional instrument (25) can be alternatively fixed to the fibroscope part (11) by means of the holding device or can be released from the fibroscope part (11) to allow a relative movement along the longitudinal direction of the additional instrument (25) and of the fibroscope part (11).

3. An endoscope in accordance with any one of the preceding claims, **characterised in that** the holding device has at the distal end of the fibroscope part (11), for the acceptance of the additional instrument (25), a loop (27) to engage around the additional instrument (25) and whose free length can preferably be actively modified.

4. An endoscope in accordance with claim 3, **characterised in that** the loop (27) is held by fixation forceps (21) which are guided in a work passage (33, 45) provided at the fibroscope part (11); or **in that** the loop (27) projects out of a work channel (33, 45) - at both the distal and proximal ends - provided at the fibroscope part (11).

5. An endoscope in accordance with any of the preceding claims, **characterised in that** a groove (49) or a holding clamp (53) and a corresponding rail (47) or corresponding rail segments are provided as a holding device at the fibroscope part (11), on the one hand, and at the additional instrument (25), on the other hand, or vice versa.

6. An endoscope in accordance with any one of the preceding claims, **characterised in that** the counter-element is an integral part of the additional instrument (25) or of the fibroscope part (11); and/or **in that** the permanent magnet (55) is provided at a plurality of sections of the fibroscope part (11) or of the additional instrument; and/in or that the permanent magnet (55) and/or the counter-element have a covering (57) of plastic.

7. An endoscope in accordance with any one of the preceding claims, **characterised in that** a catch element (61) and a hook device (59) are provided as the holding device at the fibroscope part (11), on the one hand and at the additional instrument (25) on the other hand, or vice versa.

8. An endoscope in accordance with claim 7, **characterised in that** the catch element (61) and/or the hook device (59) are each provided at the distal end of the fibroscope part (11) or of the additional instrument (25) respectively; and/or **in that** the catch element (61) is formed by a laterally projecting button lug and the hook device (59) by a laterally projecting undercutting lug; and/or **in that** the catch element (61) and/or the hook device (59) are formed in a manner flattened towards the proximal end of the endoscope.

9. An endoscope in accordance with any one of the preceding claims, **characterised in that** at least one fastening hoop (29) is provided along the insertion section (13) of the endoscope and spaced apart-from its distal end for the guidance of the additional instrument (25).

10. An endoscope in accordance with any one of the preceding claims, **characterised in that** a jacket hose (31) or a side cover (45) is provided at the fibroscope part (11) as a holding device for the receiving of the additional instrument (25), said jacket hose (31) or side cover (45) extending along the whole insertion section or along a part of the insertion section (13) of the endoscope.

11. An endoscope in accordance with any one of the previous claims, **characterised in that** the jacket hose (31) surrounds both the fibroscope part (11) and the additional instrument (25) and/or is elastically formed with respect to its diameter; and/or **in that** the jacket hose (31) or the side cover (45) is formed displaceably with respect to the fibroscope part (11), in particular due to a rail-groove connection (Fig. 15b).

12. An endoscope in accordance with any one of the preceding claims, **characterised in that** the cross-section (43) of the insertion section (13) is matched to the body orifice (41); and/or **in that** the endoscope is formed as a pharingo-oesophago-gastroscope for the examination of the pharynx, oesophagus and/or stomach, wherein the cross-section (43) of the insertion section (13) is matched to the cross-section of a human meatus of the nose (41).

13. An endoscope in accordance with any one of the preceding claims, **characterised in that** the cross-section dimension of the insertion section (13) is larger in one direction, in particular larger at least by a factor of 1.5, than in a direction orthogonal thereto; and/or **in that** the cross-section (43) of the insertion section (13) corresponds to an isosceles triangle or to a mirror-symmetrical trapezium, each with rounded corners and preferably with a base length of at most approximately 3.5 mm.

14. An endoscope in accordance with any one of the preceding claims, **characterised in that** the additional instrument (25) is formed by biopsy forceps, an aspirator/injector probe, a pH probe, a pressure measuring instrument and/or a Bilitec measuring probe; and/or **in that** the maximum cross-section dimension of the additional instrument (25) amounts to at most approximately 3 mm and preferably at most approximately 2 mm.

15. An endoscope in accordance with any one of the preceding claims, **characterised in that** the additional instrument (25) is provided laterally spaced with respect to the centre of the cross-section (43) of the insertion section (13); and/or **in that** the fibroscope part (11) and the additional instrument (25) are displaceable relative to-one another by a length of up to approximately 5 cm or of up to approximately 35 cm along their longitudinal directions.

16. An endoscope in accordance with any one of the preceding claims, **characterised in that** the endoscope has a Bowden cable (23) by which the endoscope can be actively pivoted in the direction of its larger cross-sectional dimension; and/or **in that** at least one separate light transmission passage (17) and at least one separate image transmission passage (19) are provided as the light/image transmission passages.

## Revendications

1. Endoscope déformable, qui comprend un ou plusieurs canaux de transmission de lumière/image (17, 19) et au moins un appareil additionnel (25),
dans lequel le canal de transmission de lumière/image ou les plusieurs canaux de transmission de lumière/image (17, 19) forme(nt), en particulier conjointement avec au moins un canal de travail (33), une partie de fibroscope fermée (11),
ladite partie de fibroscope (11) étant séparable de l'appareil additionnel (25), et il est prévu un dispositif de maintien pour maintenir et/ou pour guider la partie de fibroscope (11) et l'appareil additionnel (25) l'un par rapport à l'autre,
dans lequel sont prévus à titre de dispositif de maintien sur la partie de fibroscope (11) d'une part et sur l'appareil additionnel (25) d'autre part, un aimant permanent (55), ou respectivement au moins un élément antagoniste (25) en matériau magnétique-permanent ou magnétique, ou inversement,
**caractérisé en ce que**
l'unité formée par la partie de fibroscope (11) et l'appareil additionnel (25) présente, le long d'un tronçon longitudinal (13) à introduire dans une ouverture corporelle (41) humaine ou animale (tronçon d'introduction), une section non ronde (43), et
**en ce que** la partie de fibroscope (11) et l'appareil additionnel (25) sont. déplaçables l'un par rapport à l'autre le long de leur direction longitudinale.

2. Endoscope selon la revendication 1, **caractérisé en ce que** l'appareil additionnel (25) est susceptible d'être fixé au moyen du dispositif de maintien au choix sur la partie de fibroscope (11) ou d'être libéré depuis la partie de fibroscope (11) pour permettre un mouvement relatif le long de la direction longitudinale de l'appareil additionnel (25) et de la partie de fibroscope (11).

3. Endoscope selon l'une des revendications précédentes, **caractérisé en ce que** le dispositif de maintien (11) à l'extrémité distale de la partie de fibroscope (11) pour la réception de l'appareil additionnel (25) présente une boucle (27) destinée à entourer l'appareil additionnel (25), dont la longueur libre est modifiable, de préférence de manière active.

4. Endoscope selon la revendication 3, **caractérisé en ce que**
la boucle (27) est maintenue par une pince de prise (21), laquelle est guidée dans un canal de travail (33, 45) prévu sur la partie de fibroscope (11), ou
**en ce que** la boucle (27) dépasse aussi bien à l'extrémité distale qu'à l'extrémité proximale d'un canal de travail (33, 45) prévu sur la partie de fibroscope (11), hors de celui-ci.

5. Endoscope selon l'une des revendications précédentes, **caractérisé en ce qu'**il est prévu à titre de dispositif de maintien sur la partie de fibroscope (11) d'une part et sur l'appareil additionnel (25) d'autre part une gorge (49) ou une pince de maintien (53), ou respectivement une tringle correspondante (47) ou des segments de tringle correspondants, ou inversement.

6. Endoscope selon l'une des revendications précédentes, **caractérisé en ce que**
l'élément antagoniste fait partie intégrante de l'appareil additionnel (25) ou respectivement de la partie de fibroscope, et/ou
**en ce que** l'aimant permanent (55) est prévu au niveau de plusieurs tronçons de la partie de fibroscope (11) ou respectivement de l'appareil additionnel, et/ou
**en ce que** l'aimant permanent (55) et/ou l'élément antagoniste comporte un recouvrement (57) en matière plastique.

7. Endoscope selon l'une des revendications précédentes, **caractérisé en ce qu'**il est prévu à titre de dispositif de maintien sur la partie de fibroscope (11) d'une part et sur l'appareil additionnel (25) d'autre part, un élément d'entraînement (61) et un dispositif d'accrochage (59), ou inversement.

8. Endoscope selon la revendication 7, **caractérisé en ce que**
l'élément d'entraînement (61) et/du le dispositif d'accrochage (59) sont prévus respectivement à l'extrémité distale de la partie de fibroscope (11) ou respectivement de l'appareil additionnel (25), et/ou
**en ce que** l'élément d'entraînement (61) est formé par un téton en forme de bouton en dépassement latéral et le dispositif d'accrochage (59) est formé par un talon à contre-dépouille en dépassement latéral, et/ou
**en ce que** l'élément d'entraînement (61) et/ou le dispositif d'accrochage (59) sont réalisés de façon aplatie vers l'extrémité proximale de l'endoscope.

9. Endoscope selon l'une des revendications précédentes, **caractérisé en ce que** le long du tronçon d'introduction (13) de l'endoscope et à distance de son extrémité distale est prévue au moins une boucle de fixation (29) pour le guidage de l'appareil additionnel (25).

10. Endoscope selon l'une des revendications précédentes, **caractérisé en ce qu'**il est prévu à titre de dispositif de maintien sur la partie de fibroscope (11) pour la réception de l'appareil additionnel (25) un tuyau enveloppe (31) ou une douille latérale (45), qui s'étend le long de la totalité du tronçon d'introduction ou le long d'une partie du tronçon d'introduction (13) de l'endoscope.

11. Endoscope selon la revendication 10, **caractérisé en ce que**
le tuyau enveloppe (31) entoure aussi bien la partie de fibroscope (11) que l'appareil additionnel (25) et/ou est réalisé de manière élastique pour ce qui concerne son diamètre, et/ou
**en ce que** le tuyau enveloppe (31) ou la douille latérale (45) est réalisé(e) de manière déplaçable par rapport à la partie de fibroscope (11), en particulier grâce à une liaison rail/gorge (figure 15b).

12. Endoscope selon l'une des revendications précédentes, **caractérisé en ce que**
la section (43) du tronçon d'introduction (13) est adapté à l'ouverture corporelle (41), et/ou
**en ce que** l'endoscope est réalisé sous forme d'endoscope pour le pharynx, l'oesophage et l'estomac pour l'examen de la gorge, de l'oesophage et/ou de l'estomac, la section (43) du tronçon d'introduction (13) étant adaptée à la section d'un canal nasal humain (41).

13. Endoscope selon l'une des revendications précédentes, **caractérisé en ce que**
la dimension de section transversale du tronçon d'introduction (13) dans une direction est supérieure, en particulier au moins d'un facteur 1,5, à celle dans une direction perpendiculaire à celle-ci, et/ou
**en ce que** la section (43) du tronçon d'introduction (13) correspond à un triangle isocèle ou à un trapèze à symétrie droite, avec des coins respectivement arrondis et de préférence une longueur de base maximum d'environ 3,5 mm.

14. Endoscope selon l'une des revendications précédentes, **caractérisé en ce que**
l'appareil additionnel (25) est formé par une pince de biopsie, par une sonde d'aspiration/injection, par une sonde de mesure de pH, par un appareil de mesure de pression et/ou par une sonde de mesure Bilitec, et/ou
**en ce que** la dimension de section transversale maximale de l'appareil additionnel (25) s'élève au maximum à environ 3 mm, de préférence au maximum à environ 2 mm.

15. Endoscope selon l'une des revendications précédentes, **caractérisé en ce que**
l'appareil additionnel (25) est prévu avec un écartement latéral par rapport au centre de la section (43) du tronçon d'introduction (13), et/ou
**en ce que** la partie de fibroscope (11) et l'appareil additionnel (25) sont déplaçables l'un par rapport à l'autre le long de leur direction longitudinale sur une longueur allant jusqu'à environ 5 cm ou jusqu'à environ 35 cm.

16. Endoscope selon l'une des revendications précédentes, **caractérisé en ce que**
l'endoscope comprend un système à câble Bowden (23), au moyen duquel l'endoscope peut être pivoté de façon active en direction de sa plus grande dimension de section transversale, et/ou
**en ce que** l'on prévoit à titre de canaux de transmission de lumière/image au moins un canal de transmission de lumière (17) séparé et au moins un canal de transmission d'images séparé (19).
